# EUROPEAN PATENT SPECIFICATION

(11) **EP 3 003 178 B1**
(45) Date of publication and mention of the grant of the patent: **27.04.2022**
(21) Application number: 14735759.4
(22) Date of filing: 03.06.2014
(51) Int. Cl.: A61B 17/30, A61B 1/00, A61B 1/32, A61B 17/00, A61M 29/02

(54) **VACUUM-ASSISTED PANCREATICOBILIARY CANNULATION**
VAKUUMUNTERSTÜTZTE BAUCHSPEICHELDRÜSEN-/GALLENKANÜLE
CANULATION BILIO-PANCRÉATIQUE ASSISTÉE PAR DÉPRESSION

(30) Priority: 04.06.2013 US 201361830931 P; 02.06.2014 US 201414293162
(43) Date of publication of application: 13.04.2016
(73) Proprietor: Boston Scientific Scimed, Inc., Maple Grove, MN 55311 (US)
(72) Inventor: KAPPEL, Gary, S., Acton, MA 01720 (US); NAHON, Vanessa, Brighton, MA 02135 (US); FIRSTENBERG, Laura, E., Worcester, MA 01605 (US); WINDHEUSER, James, E., Hopkinton, MA 01748 (US); BALDERRAMA, Desiree, D., Boston, MA 02130 (US); MANNION, Paul, Shrewsbury, MA 01545 (US); SIMANI, Heather, A., Dedham, MA 02026 (US); COHEN, Adam, L., Sudbury, MA 01776 (US); CROWLEY, Peter, Norfolk, MA 02056 (US)
(74) Representative: Vossius & Partner Patentanwälte Rechtsanwälte mbB
(86) International application number: PCT/US2014/040636
(87) International publication number: WO 2014/197444

(56) References cited:
- WO-A1-2008/102933
- US-A1- 2006 235 269
- US-A1- 2008 058 591
- US-B1- 6 174 307

## Description

### TECHNICAL FIELD

Various embodiments of the present disclosure relate generally to medical devices and related exemplary methods of use thereof. More specifically, the present disclosure relates to devices and exemplary methods for accessing the pancreaticobiliary system, e.g., to examine, diagnose, and/or treat a condition of the pancreatic duct or the bile duct.

### BACKGROUND

Access to the pancreaticobiliary system is required to diagnose and/or treat a variety of conditions, including tumors, gallstones, infection, sclerosis, and pseudocysts. One method of gaining access is via endoscopic retrograde cholangiopancreatography (ERCP), in which a side-viewing endoscope is passed down the esophagus, through the stomach, and into the duodenum where the duodenal papilla leading into the pancreatic and bile ducts may be visualized. In ERCP, tools such as sphincterotomes are passed through the working channel of the scope to gain access to the papilla, e.g., to investigate potential obstruction or inflammation of the pancreatic or bile ducts. Fluoroscopic contrast may be injected into either duct and X-ray images taken to determine the presence and location of strictures or stones.

Cannulation of either the bile duct or the pancreatic duct is a significant challenge in ERCP procedures. Factors that may complicate insertion into the papilla include sphincter orientation, floppy intraductal segments, biliary/pancreatic take-off levels, and the presence of stones or strictures. Difficult cannulations carry a high risk of perforation or other damage to tissue. For example, one technique physicians use to cannulate the papilla is to identify a bile trail, e.g., by pushing against the ampulla to encourage bile from the duct. Prolonged probing, however, may lead to inflammation of the papilla and adverse effects for the patient.

Complications also may arise when the duct accessed first is not the duct desired for the procedure. When biliary access is desired, for example, a physician first may gain access to the pancreatic duct, e.g., via a guidewire. The physician then would have to remove the wire and attempt cannulation again. The pancreatic duct may be entered unintentionally several more times before access to the bile duct is finally achieved. These multiple pancreatic injections can irritate the tissue of the pancreatic duct and cause post- ERCP complications such as pancreatitis.

US 2008/058591 A1 relates to tissue visualization devices and variations thereof. Such devices may utilize a variety of methods for facilitating clearing of the device of opaque bodily fluids and sealing between the device and the underlying tissue surface. Additionally, methods and devices for enhancing navigation of the device through a patient body are also described. WO 2008/102933 discloses an endoscope tube having a distal vacuum cap.

Thus, there remains a need for alternative devices for accessing the pancreaticobiliary system in order to improve efficacy of medical treatment and increase patient safety.

### SUMMARY OF THE DISCLOSURE

The invention for which protection is sought is defined by the independent claim. The dependent claims concern particular embodiments. Methods do not form part of the claimed invention.

The present disclosure includes devices and exemplary methods of use thereof for cannulating the papilla, such as during an ERCP procedure.

The present disclosure includes a medical device comprising: a tube having a proximal end, a distal end, and at least one channel extending therebetween, the at least one channel in communication with a side aperture at the distal end of the tube; and a cap disposed on the distal end of the tube, the cap including an opening in communication with the aperture and an expandable appendage disposed around the opening, wherein the appendage is configured to form a seal with a tissue surface.

Embodiments of the present disclosure may include one or more of the following features: the appendage may include an elastomeric material; a distal most surface of the appendage includes a surface feature to grip the tissue surface; the cap may have a retracted configuration for moving the medical device along a body lumen and an expanded, conical configuration for engaging the appendage with the tissue surface; the appendage may include doors that pivot outward from the opening to engage the tissue surface; the cap may be transparent; a distal most surface of the appendage may include a deformable portion capable of forming a seal with the tissue surface; the cap may be removable from the tube; the medical device may comprise at least one treatment instrument slidably disposed in the channel; the appendage may include at least one inflatable member; or the appendage may include a flexible membrane and a plurality of inflatable members attached to the membrane, wherein the inflatable members extend radially outward from the opening to expand the membrane into the conical shape.

The present disclosure further includes an exemplary method of accessing the pancreaticobiliary system, the method comprising: introducing a suction device into a body lumen, the suction device having a retracted configuration for moving along the body lumen; deploying the suction device into an expanded configuration to form a seal with the tissue surface, wherein the tissue surface includes a papilla; and applying suction with the suction device.

Embodiments of the present disclosure may include one or more of the following features: the suction device may include a tube and a cap disposed on a distal end of the tube, the cap including an expandable appendage for transitioning between the retracted configuration and the expanded configuration; the exemplary method may comprise introducing a guidewire into at least a portion of the papilla; and advancing an instrument along the guidewire and through the papilla; the exemplary method may comprise interrupting the suction to draw the guidewire into the papilla before advancing the instrument along the guidewire; applying suction may cause bile to exit through the papilla; the instrument may be a sphincterotome, the exemplary method further comprising cutting at least a portion of the tissue surface with a cutting wire of the sphincterotome; the instrument may be advanced through the papilla into a bile duct or a pancreatic duct; the exemplary method may comprise inflating an inflatable portion of the suction device; or the suction device may include an end cap, the exemplary method further comprising placing the end cap over a distal end of an endoscope.

### BRIEF DESCRIPTION OF THE FIGURES

The accompanying drawings, which are incorporated in and constitute a part of this specification, illustrate various exemplary embodiments and together with the description, serve to explain the principles of the disclosed embodiments.
FIG. 1 shows anatomical features of the pancreaticobiliary system.
FIGS. 2A-2C illustrate an exemplary method of accessing the pancreaticobiliary system, in accordance with the present disclosure.
FIGS. 3A-3E illustrate an exemplary method of accessing the pancreaticobiliary system, in accordance with the present disclosure.
FIGS. 4A-4B show a device, in accordance with the present disclosure.
FIGS. 5A-5B show a device, in accordance with the present disclosure.
FIGS. 6A-6B show a device, in accordance with the present disclosure.
FIGS. 7A-7B show a device, in accordance with the present disclosure.
FIGS 8A-8B show a device, in accordance with the present disclosure.
FIG. 9 shows a device, in accordance with the present disclosure.

### DETAILED DESCRIPTION

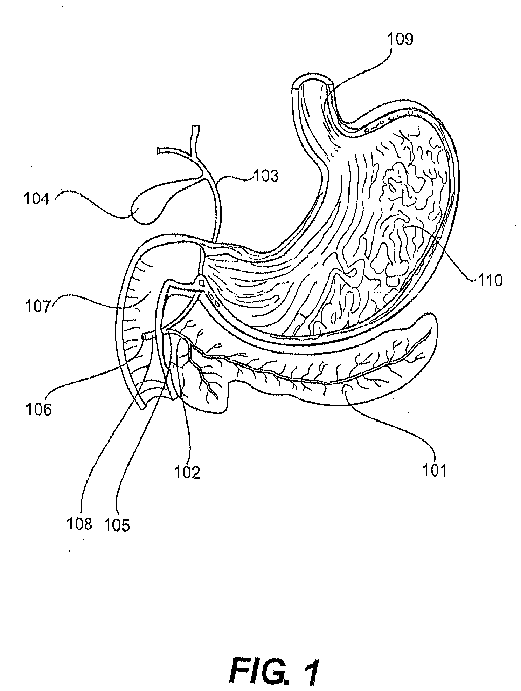

The pancreaticobiliary system, illustrated in FIG. 1, includes the pancreas (101), the pancreatic duct (102), the common bile or biliary duct (103), and the gallbladder (104). The pancreatic and biliary ducts join at the hepatopancreatic ampulla (105) (also known as the ampulla of Vader), which lies just behind the major duodenal papilla (106). The papilla (106) is a small opening that leads into the duodenum (107) to allow for the release of pancreatic juice and bile into the duodenum to aid in digestion. Smooth muscle of the hepatopancreatic sphincter (108) (also known as the sphincter of Oddi) regulates flow of pancreatic juice and bile into the duodenum. The minor duodenal papilla (not shown) is a separate small opening in the duodenum, upstream of the major papilla (106), that leads into the accessory pancreatic duct. The minor papilla is usually nonfunctional (i.e., does not release pancreatic juice into the duodenum) and may be absent, for example in patients lacking an accessory pancreatic duct. While the present disclosure generally relates to the major duodenal papilla (referred to herein simply as "papilla"), it is understood that the present disclosure also may be useful in accessing the minor duodenal papilla.

Referring again to FIG. 1, in an ERCP procedure, an endoscope may be passed down the esophagus (109), through the stomach (110), and into the duodenum (107) to gain access to the pancreatic duct (102) and/or bile duct (103) via the papilla (106). The passageway leading from the papilla (106) towards the pancreatic duct (102) and bile duct (103) tends to be tortuous and difficult to navigate, however, e.g., via a guidewire, catheter, or other medical device. In some patients, the papilla may also be obscured from view by a diverticulum. A physician may make several unsuccessful attempts at cannulation, increasing the risk of injury to the patient, before access is achieved.

According to embodiments of the present disclosure, negative pressure or suction may be applied to the papilla, e.g., duodenal tissue surrounding the papilla, to facilitate cannulation. For example, a device may be used to suction or exert a pulling force on the duodenal surface to straighten tissue folds and/or smooth muscle bands, e.g., of the sphincter, ampulla, bile duct, and/or pancreatic duct. Smoothing tissue and/or muscles surrounding the papilla may allow for better visualization of the papilla and enable more direct entry therein. Suction also may draw bile from the bile duct, providing a visible bile trail to assist in locating the papilla. Identifying a bile trail may also enable a physician to distinguish the bile duct from the pancreatic duct, thus facilitating the introduction of a guidewire, cannula, catheter, or other medical device into the desired duct, e.g., for visualization and/or treatment. Suction may also be used to remove material, e.g., from the pancreaticobiliary system, or as a pseudo cyst drainage system.

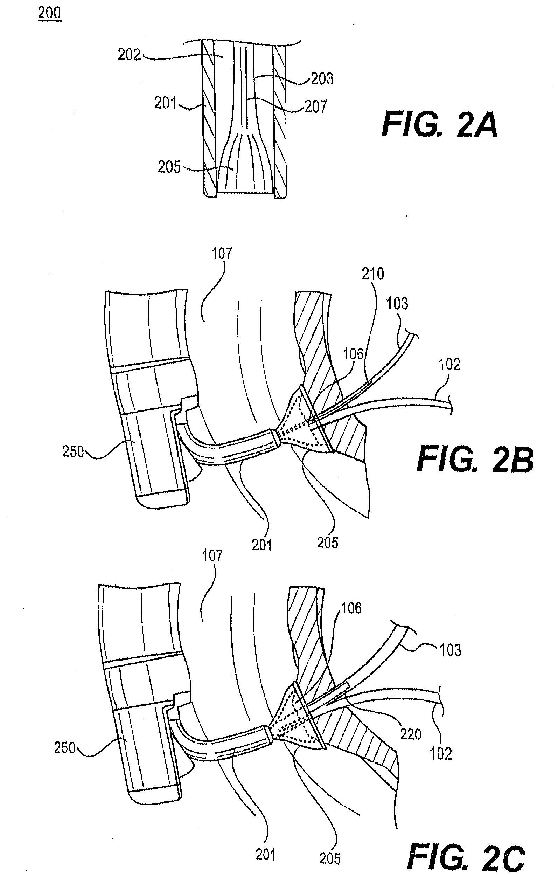

In some embodiments of the present disclosure, a device configured to apply suction maybe introduced into the working channel of an endoscope, e.g., a side-viewing endoscope, to reach the papilla. According to one embodiment shown in FIGS. 2A-2C, the device (200) comprises an elongate body (201) having a proximal end, a distal end, and one or more lumens (202) extending therebetween. The device (200) further comprises an inner tubular member (203) that is slidable within the lumen (202), wherein a distal end of the inner tubular member (203) includes a suction cup (205). The suction cup (205) may be an integral part of the inner tubular member (203), or may be a separate component that is fixedly or removably attached to the distal end of the inner tubular member (203).

In some embodiments, the suction cup (205) may be collapsible, e.g., having a collapsed configuration and an expanded configuration. FIG. 2A shows suction cup (205) in a collapsed configuration, constrained within the elongate body (201). Moving the suction cup (205) in a distal direction beyond the end of the elongate body (201) deploys the suction cup (205) into an expanded configuration having a generally conical shape as shown in FIGS. 2B-2C. The suction cup (205) may be moved in a proximal direction back within the elongate body (201), e.g., into a collapsed configuration. In some embodiments of the present disclosure, however, the suction cup may not be collapsible and may maintain a conical shape.

While FIG. 2A illustrates one mechanism for deploying the suction cup (205), e.g., a self-expansion, other mcchanisms may be used. The suction cup (205) may be deployed or expanded via a push- or pull-wire, or a spring, for example, or other suitable mechanism. For example, a sphincterotome or other instrument may assist in deploying and/or retracting the suction cup (205). In some embodiments, the suction cup (205) may be an integral part of the distal end of the elongate body (201). In other embodiments, the suction cup (205) may be a separate component that is fixedly or removably attached to the distal end of the elongate body (201).

The suction cup (205) may have a conical or funnel shape (e.g., generally circular or oval cross-section) as shown in FIGS. 2A-2C, but may have any other shape appropriate for contacting a surface and applying suction. The device (200) may include one or more materials that provide flexibility as well as columnar integrity to ensure that the device (200) does not collapse when suction or vacuum is applied. The suction cup (205) and elongate body (201) may be formed from any suitable biocompatible materials, including one or more flexible, deformable, elastomeric, or expandable materials. Non-limiting examples of materials that may be used for the suction cup (205) and/or elongate body (201) include silicone, rubber, metals, plastics, and polymers or polymer mixtures (e.g., polyethylene, polyurethane, polycarbonate, fluoropolymers, copolymers, etc.). The suction cup (205) may include one or more coatings, such as a lubricious coating.

The suction cup (205) may provide a greater field of view and/or greater region of access when placed against a tissue surface. In some embodiments, the distal most surface of the suction cup (205) may include a deformable portion such as, e.g., a layer of silicone or other deformable material to provide for more uniform contact with a tissue surface. The deformable portion may include an inflatable member such as a balloon that is capable of conforming to the contour of the tissue surface. In some embodiments, the distal most surface of the suction cup may include one or more surface features to grip the tissue surface. The distal most surface of the suction cup may include, for example, ridges, grooves, barbs, hooks, and/or a coarse material to grip tissue and enhance friction when contacting the tissue surface.

In at least some embodiments, the suction cup comprises a flexible membrane, e.g., a non-permeable or semi-permeable membrane. The membrane may include one or more support members such as, e.g., support arms extending radially outward or circular supports embedded within or otherwise attached to the circumference of the membrane. The support members may comprise a rigid material such as, e.g., metal or plastic, to maintain a predefined shape. In a collapsed configuration as shown in FIG. 2A, for example, support arms may be drawn close together in a confined space and expand into a conical or funnel shape together with the membrane in an expanded configuration as shown in FIGS. 2B-2C.

A split catheter tip may also be used according to some embodiments, wherein split or divided portions of a catheter tip may be molded into the desired shape, e.g., a concical shape, and coated with a web of material. The split catheter may be deployed similarly to the suction cup illustrated in FIG. 2A, e.g., by compressing the catheter tip, loading it into an endoscope, and deploying the tip into an expanded shape by advancing the catheter tip distally outside a sheath. The catheter tip may be deployed by an alternative mechanism such as, e.g., pull-wire, spring, or other suitable mechanism.

In another embodiment, the suction cup may include a ring- or donut-shaped inflatable member. The inflatable member may be expanded to form a conical or funnel shape, e.g., by pressing the inflatable member from a proximal direction via a sheath.

In yet another embodiment, access to the pancreaticobiliary system may be facilitated by using a diverted catheter to apply pressure against the ampullary wall while a catheter or wire is advanced into the desired duct, e.g., a third hand concept. The shape of the third hand may be a halo hoop that encircles the ampulla (105), for example, or a flip up paddle that pushes one side but may be rotated around the circumference of the ampulla (105), or like fingers similar to a the feet of a lunar lander.

According to some embodiments of the present disclosure, the device (200) may be used for a medical procedure, such as an ERCP procedure. As shown in FIGS. 2B-2C, the device (200) may be introduced into the working channel of an endoscope (250) to reach the papilla (106) through an aperture of the endoscope (250). The endoscope (250) may include a proximal end and a distal end, the working channel extending therebetween, wherein the aperture is located at the distal end of the endoscope (250). In at least some embodiments, the endoscope (250) maybe a side-viewing endoscope, i.e., having a side aperture at the distal end, as shown in FIGS. 2B-2C. The side-viewing endoscope (250) may include a positioning mechanism such as, e.g., a ramp, elevator, or other feature to assist in deploying and/or orienting the device (200) towards the papilla (106). The endoscope (250) may also include one or more proximal ports for receiving instruments, such as device (200) in the working channel. In some embodiments, the endoscope (250) may supply suction and/or inflation air, or an inflation tube or vacuum/suction tube may be attached to the endoscope (250), e.g., via a removable adhesive strip or other suitable material or mechanism to provide inflation air and/or suction capability.

The suction cup (205) may be deployed, e.g., by moving the inner tubular member (203) distally through an opening in the elongate body (201) as shown in FIG. 2A, to bring the suction cup (205) into an expanded configuration. The suction cup (205) may brought into contact with the duodenal surface surrounding the papilla (106). Suction may be applied through a lumen (207) in communication with the suction cup (205) to apply negative pressure to smooth tissue folds around the papilla (106) and/or draw bile from the bile duct (103) to assist in identification and/or cannulation of the papilla (106).

A guidewire (210) may pass through the device (200) via the lumen (207) used for suction or another lumen in communication with the suction cup (205). While FIGS. 2A-2C show a single lumen (207), in other embodiments, the device (200) may include two or more lumens to provide separate channels for applying suction and passage of a guidewire. The guidewire (210) may be advanced through the suction cup (205) to enter the papilla (106) as shown in FIG. 2B. Guidewires are available in a variety of diameters, e.g., ranging from about 0.018" to about 0.035" outer diameter, and typically include a solid metallic core with an applied coating. The coating may have markings for visual indicators, e.g., radiopaque markers, and may provide a lubricious surface for a catheter passed over the wire. The guidewire (210) may be of sufficient length to allow passage through the working channel of the endoscope (250), and the tip of the guidewire (210) maybe tapered and/or constructed of a softer material to promote cannulation and minimize trauma to the patient. The guidewire (210) may be selectively introduced into the pancreatic duct (102) or the bile duct (103). For example, a physician may distinguish the bile duct from the pancreatic duct visually with the assistance of a visible bile trail, and advance the guidewire (210) into the desired duct for examination.

The guidewire (210) may allow for exchange of a catheter (220) or other treatment instrument introduced through the device (200) as shown in FIG. 2C. While FIGS. 2B-2C illustrate insertion of a guidewire (210) during cannulation of the papilla (106), in some embodiments cannulation may be achieved without the use of a guidewire (210). The catheter (220) may be flexible, and may include a tapered tip, typically ranging from about 3 Fr to about 6 Fr in diameter, to ease cannulation of the papilla. The catheter (220) may include one or more lumens, e.g., for receiving guidewire (210) and injecting a contrast agent for fluoroscopy or other imaging analysis. The catheter (220) may be steerable, e.g., to control movement of the distal end of the catheter (220). In some embodiments, the distal end of the catheter (220) may be deflected in one or more directions to align the catheter tip with the papilla (106).

In some embodiments , the catheter (220) is a sphincterotome. For example, the sphincterotome may include an electrosurgical cutting wire at the distal end to enable deflection of the sphincterotome tip and to provide transmission of high frequency electrical current to incise the sphincter (108). In addition to aligning the sphincterotome with the papilla (106), deflection of the tip also may help to maintain contact with tissue of the ampulla (105) during incision. The physician may incise the sphincter to gain access to the pancreaticobiliary system according to some embodiments of the present disclosure, but incision may not be necessary.

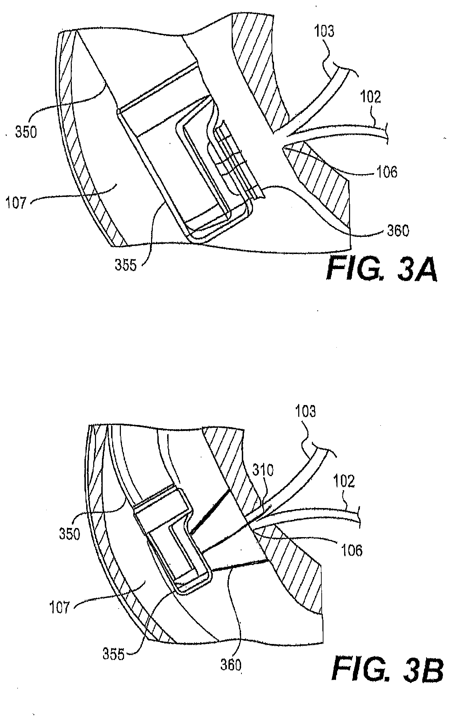

As an alternative, or in addition to use of a device including a suction cup as in FIGS. 2A-2C, the distal end of the endoscope may be configured to apply suction, e.g., via a cap configured to contact the wall of the duodenum. While the following describes using cap (355) to apply suction, the cap (355) may also be configured for insufflation, e.g., to distend the area around the papilla (106) to help in identifying the opening. Insufflation may be applied via the same channels used for suction, or additional or other channels. FIGS. 3A-3D illustrate an embodiment comprising a cap (355) that fits over the end of an endoscope (350). The cap (355) may be fixedly attached to the endoscope (350), or also may be removable and capable of sliding over the distal end of the endoscope (350), e.g., to form a friction fit. The cap (355) includes an appendage (360) configured for engaging with a tissue surface. The appendage (360) may form a conical or funnel shape (e.g., generally circular or oval cross-section) as shown in FIGS. 3A-3C, but may have any other shape appropriate for contacting a surface and applying suction. In some embodiments, for example, the appendage (360) may have a rectangular cross-section. The cap (355) may provide a greater field of view and/or greater region of access when placed against a tissue surface.

In some embodiments, the appendage (360) of the cap (355) may include a purse-string feature to vary a cross section of the appendage (360). For example, the appendage (360) may form a conical or funnel shape with a purse-string feature at the distal end of the funnel to allow for widening or narrowing the diameter of the funnel in contact with the tissue surface. The purse-string feature may help to grasp and manipulate tissue, and may also help to guide an instrument into position, e.g., to cannulate the papilla. The purse string may also be used to apply suction and enclose a portion of tissue, e.g., for removal via a snare. In such an embodiment, a purse-string may be looped around the distal surface of the appendage (360), and fed through a lumen of the endoscope (350) to the proximal end. A user may pull on the purse-string at the proximal end of the endoscope (350) to reduce the size, e.g., diameter, of the opening formed by appendage (360).

The cap (355) may include a recessed area or window. In some embodiments, the window may include integrated cautery wire capability for cutting and/or cauterizing tissue pulled into the window via suction.

The cap (355) has a retracted configuration, e.g., for introducing the endoscope (350) into the duodenum as shown in FIG. 3A, and an expanded configuration, e.g., for engaging with the duodenal wall to contact the tissue surface and form a seal with the tissue surrounding the papilla (106) as shown in FIGS. 3B-3D. The retracted configuration may be a bellows shape. The cap (355) may be deployed from the retracted configuration to the expanded configuration via a pull wire, push wire, spring, or other suitable mechanism. The cap may include one or more support members, e.g., as discussed above in connection with suction cup (205), to support a membrane in a retracted configuration and/or an expanded configuration.

Any of the materials and/or features described above in connection to the suction cup (e.g., suction cup (205) of FIGS. 2A-2C) may be used for the cap (355). For example, the cap (355) may be formed of one or more flexible and/or rigid materials including, e.g., silicone, rubber, metals, plastics, and polymers or polymer mixtures. In some embodiments, the cap (355) includes an elastomeric material. In some embodiments, the cap may be transparent, e.g., to permit imaging and lighting functions of the endoscope (350).

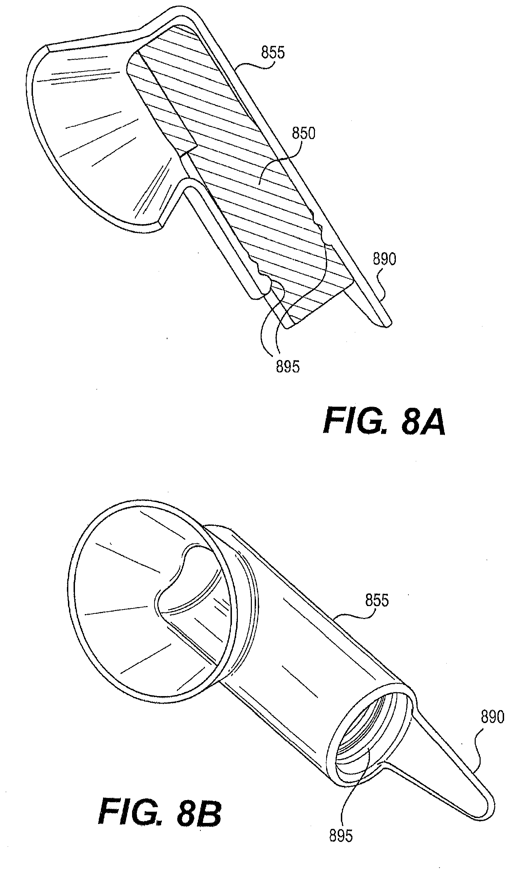

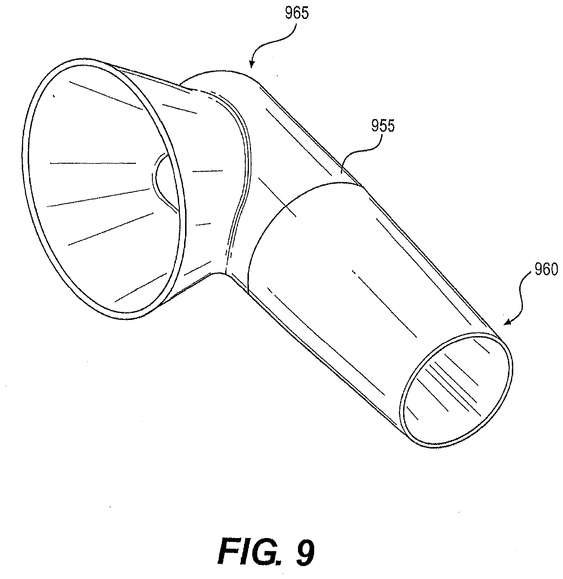

The cap (355) may have a closed distal end. For example, in some embodiments, the endoscope (350) and cap (355) are configured such that the only openings include the face of the appendage (360) and an opening at the proximal end of the endoscope (350) to allow for the cap (355) to fit over the endoscope (350). The proximal end of the cap (355) may include one or more elastic bands to secure the cap (355) over the endoscope (350), such as to provide a seal at the proximal end of the cap (355) so that suction is applied only at the face of the appendage (360). See also FIGS. 8A-8B and 9 below. The cap (355) may also include an open distal end to be deployed proximally, e.g., by sliding the cap (355) down the length of the endoscope into position distally, wherein the cap (355) includes a closing, sealing feature, e.g., a purse string or other mechanical mechanism, to close the distal end.

The cap (355) may be sufficiently collapsible, flexible, and tearable such that it may be pulled through a working channel of the endoscope (350), if desired, after placement of a guidewire or other cannulation of the papilla (106), pancreactic duct (102), and/or bile duct (103). This may be done by, e.g., extending a grasper through an endoscope working channel, grasping the cap (355), and pulling it back through the channel.

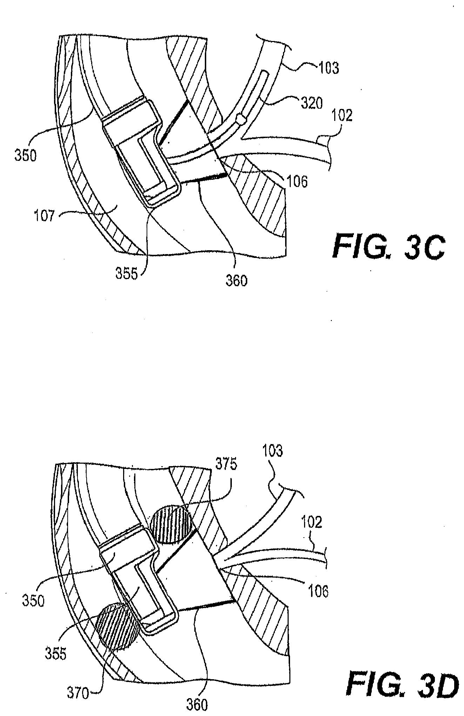

The endoscope (350) and cap (355) according to the present disclosure may be used for a medical procedure, e.g., an ERCP procedure, as described above in connection to FIGS. 2A-2C. Thus, referring to FIGS. 3B-3C, the cap (355) may brought into contact with the duodenal surface surrounding the papilla (106) and suction applied to smooth tissue folds and/or muscles around the papilla (106), ampulla (105) and/or sphincter (108). Suction may also draw bile from the bile duct (103), providing a visible bile trail. A guidewire (310) may be introduced into the papilla (106) to assist in cannulation as shown in FIG. 3B, followed by a catheter (320) such as a sphincterotome as shown in FIG. 3C, or other treatment instrument over the guidewire (310) for cannulation and/or examination, diagnosis, treatment, etc., within the pancreatic duct (102) and/or bile duct (103). As noted above, a guidewire (310) may not be necessary for cannulation.

In some embodiments, the cap (355) may include an inflatable member to assist in securing the cap (355) against the tissue surface. As shown in FIG. 3D, for example, the cap (355) may include an inflatable member such as a balloon (370) on the back side of the cap (355) directly opposite the appendage (360), wherein inflating the balloon (370) causes the balloon (370) to press against the duodenal wall opposite the papilla (106) and create forward pressure on the cap (355) to contact the tissue surface surrounding the papilla (106). In addition or alternatively, the cap (355) may include an inflatable member such as balloon (375) on the same side as the appendage (360), e.g., just below the appendage (360) (i.e., proximal to the appendage (360)), to press against the duodenal wall of the papilla (106) to create space between the cap (355) and the papilla (106). Embodiments of cap (355) may include one or both of these balloons (370, 375). To inflate the balloons (370, 375), a channel may extend through the endoscope (350) to provide inflation fluid to the cap (355) and its balloons (370, 375).

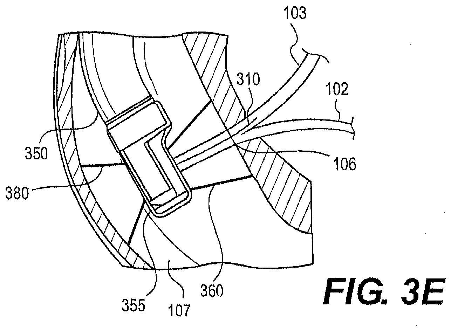

In some embodiments, the cap (355) may provide more than one suction area or channel, e.g., for applying suction to two or more tissue surfaces independently or in combination with each other, and/or for guiding various instruments. For example, the cap (355) may include a suction area opposite the appendage (360), such as a second appendage (380) as illustrated in FIG. 3E, for applying suction against a tissue surface opposite the papilla (106). The second appendage (380) may help to maintain the position of the endoscope (350) and/or the main or first appendage (360) with respect to the papilla (106), or may draw tissue tight to create traction, smooth tissue folds, create additional working space, or help to open up the papilla (106). The second appendage may connect to a suction channel between the cap (355) and the endoscope (350), e.g., a suction channel external to the endoscope (350), or may connect to a working channel within the endoscope (350). In some embodiments, suction may be applied first to the tissue surface surrounding the papilla (106) via the first appendage (360), followed by suction applied to a tissue surface of the duodenum (107) opposite the papilla (106) via second appendage (380) to maintain the position of the first appendage (160). Alternatively, suction may first be applied to a tissue surface opposite the papilla (106) via second appendage (380), e.g., to draw suction and help smooth tissue surrounding the papilla (106), followed by suction applied to the papilla surface. While the second appendage (380) is illustrated as directly opposite the first appendage (360) in FIG. 3E, the second appendage (360) may be located anywhere along the cap (355), such as adjacent, above, below, or at an angle with respect to the first appendage (360). In some embodiments, the cap (355) may include one or more working channels to guide different instruments to an area of interest, such as the papilla (106) and/or tissue around the papilla (106).

Referring to FIG. 3B, a guidewire (310) may be advanced through the working channel of the endoscope (350), e.g., via a lumen of a catheter introduced into the working channel, through the cap to enter the papilla (106). A sphincterotome (320) or other cannulation catheter or treatment instrument may slide over the guidewire (310) to cannulate the papilla (106) as shown in FIG. 3C. The sphincterotome may be used to incise the sphincter (108) as described above, for example, and may also be used to inject contrast into the bile duct (103) and/or pancreatic duct (102) for fluoroscopy or other imaging analysis.

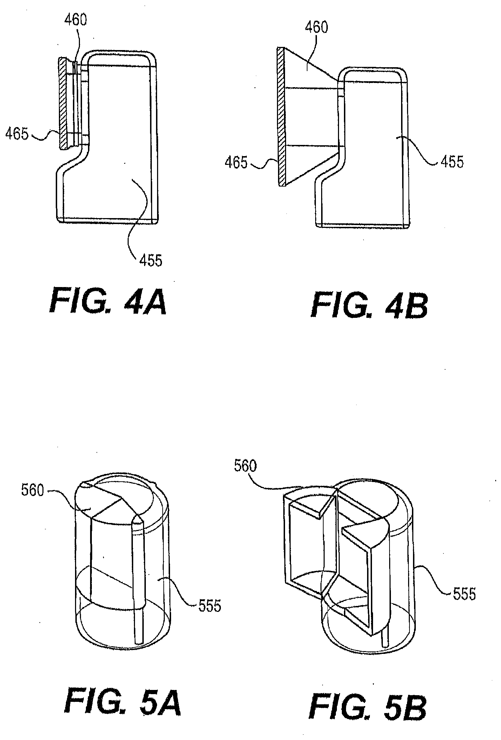

FIGS. 4A and 4B illustrate similar retracted and expanded configurations, respectively, of a cap (455) including an expandable appendage (460), wherein the expandable appendage includes a feature (465) at the distal end of the appendage (460) configured to interface with the tissue surface. In some embodiments, for example, the feature (465) may comprise a deformable material or inflatable member to adapt to the contour of the tissue surface. In other embodiments, the feature (465) may comprise a rigid material such as a metal wire or plastic ring. For example, a rigid material at or near the distal end of the appendage (460) may be used to apply pressure against the tissue surface, e.g., to spread or smooth tissue. Further, the feature (465) may include a rigid material to act as a tissue stop, e.g., to prevent tissue from being drawn into the funnel of the appendage (460) by suction. The feature (465) may be continuous, e.g., covering the entire distal end circumference of the appendage (460), or may include one or more discrete portions. In some embodiments, the distal edge of the appendage (460) or the feature (465) may include one or more holes to help release or reduce the amount of suction. In some embodiments, the appendage (460) or the feature (465) may have a scalloped or thinned edge, e.g., to help adapt to the contour of the tissue surface and accommodate an irregular surface to the tissue.

In some embodiments, the feature (465) may be used to deploy the appendage (460) into an expanded configuration. For example, the appendage (460) may include a flexible or thin film material with a distal end feature (465) comprising a rigid material such as a metal or plastic ring or wire. The appendage (460) may be deployed into an expanded configuration as shown in FIG. 4B via a spring or snare-like mechanism coupled to the rigid material of the feature (465).

In some embodiments, the feature (465) may comprise a metal or plastic ring that includes one or more metal or plastic strips or wires across a diameter of the ring and parallel to the longitudinal axis of the cap (455), i.e., parallel to the longitudinal axis of the endoscope. The wires may be concave and curved towards the face of the endoscope, allowing tissue to be partially drawn into the appendage (460) but not far enough to interfere with the field of view or field of access. In other embodiments, the feature (465) may include two or more concentric rings with radial arms connecting the rings to each other and/or to the main body of the cap (455), similar to the support arms described above.

In another embodiment shown in FIGS. 5A-5B, the cap (555) includes as an appendage one or more doors (560) configured to pivot or swing open and engage with a tissue surface. Each of the doors (560) may pivot along an axis parallel to the central longitudinal axis of the cap (555) as shown in FIGS. 5A-5B, or may also open along another direction, e.g., along an axis perpendicular or otherwise offset from the longitudinal axis. In some embodiments, the doors (560) may slide open rather than pivot open. The edge(s) of each door (560) may include a deformable or elastomeric material, e.g., to form a seal between the doors (560) in a retracted (i.e., closed) configuration and to adapt to the tissue surface in an expanded (i.e., open) configuration. Doors (560) may be opened and closed via a pull wire, push rod, or other suitable mechanism extending through the cap and a channel of the endoscope.

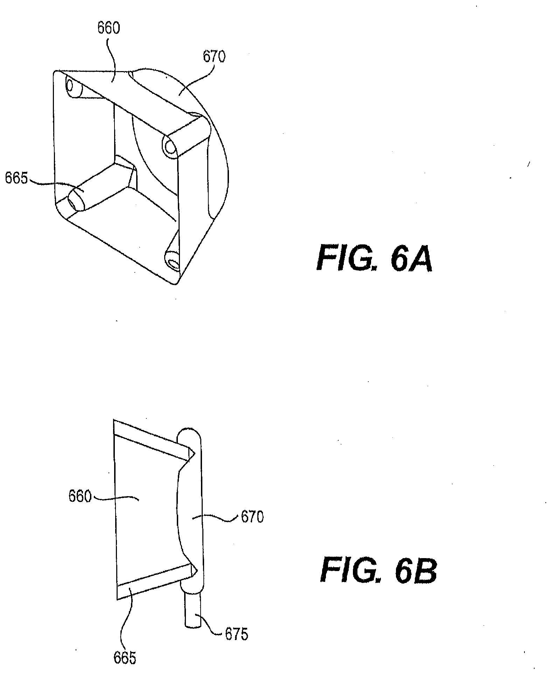

FIGS. 6A-6B illustrate yet another embodiment of a device comprising a plurality of inflatable members (665) attached to a membrane (660). The membrane (660) may comprise a flexible or elastomeric material such that upon inflation, the inflatable members (665) may expand radially outward to expand or stretch the membrane (660) to form a conical or funnel shape for engaging with the duodenal wall. The embodiment shown in FIGS. 6A-6B may be included in a device disposed in the working channel of an endoscope, e.g., a suction cup as discussed above, or may also comprise an appendage of an endoscope cap. In an embodiment shown in FIGS. 6A-6B, for example, the device includes four inflatable members (665), each of which is in fluid communication with an inflatable ring (670), wherein at least one inflatable member (665) receives an inflation fluid via any suitable mechanism. For example, a suction port 675 can connect to an inflation lumen extending through the endoscope to the proximal end, to receive inflation fluid to inflate the inflatable ring (670) and inflatable members (665), and expand the membrane.

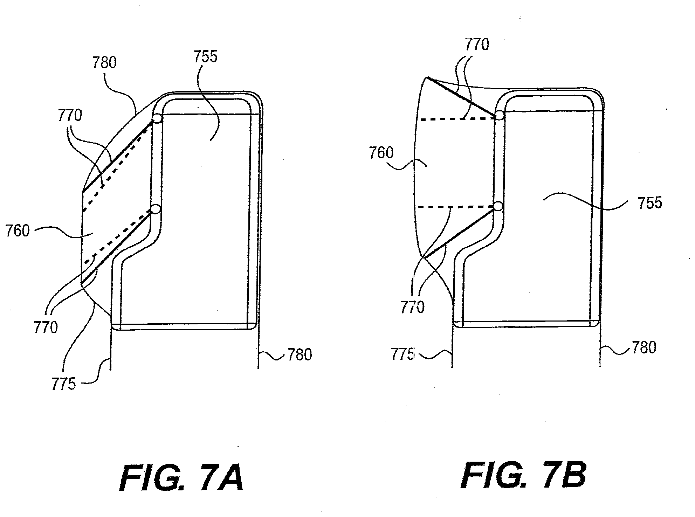

FIGS. 7A-7B illustrate another embodiment, comprising a cap (755) and appendage (760) that includes a flexible material and one or more support arms (770). Support arms (770) may include a rigid material, and may pivot with respect to the main body of the cap (755) by manipulating a push or pull wire (775), (780). As shown in FIG. 7A, pulling wire (775) in a proximal direction may cause support arms (770) to pivot downward, thus bringing the appendage (760) into a retracted and more streamlined configuration, e.g., to facilitate introducing and/or withdrawing the endoscope from the body. A second wire (780) may remain slack, or may have sufficient rigidity to push the appendage (760) downward into the retracted configuration. Tension on wire (775) may be released and/or tension on wire (780) may be increased to expand the appendage (760), e.g., into a conical shape as shown in FIG. 7B. Wire (775) may also have sufficient rigidity to pivot support arms (770) upward into the expanded configuration. In some embodiments, the distal end of the cap (755) may be configured to assist in advancing and withdrawing the cap (755) within the body. For example, the distal end of the cap (755) may include an extension, such as a pointed cone ribs extension. In some embodiments, a sphincterotome or other instrument may assist in deploying and/or retracting the appendage (760).

While FIGS. 7A-7B illustrate an embodiment with support arms, other embodiments may not include support arms, wherein one or more pull or push wires connect to the flexible material of the appendage (760) to expand and retract the appendage (760). Further, in some embodiments push or pull wires (775), (780) may be manipulated to pivot support arms (770) toward each other, e.g., to close the opening of the appendage (760) to provide a retracted and more streamlined configuration.

It should be noted that while FIGS. 3-7 illustrate both a retracted configuration and an expanded configuration of the cap, in some embodiments, not in accordance with the present claimed invention, the cap may have a single configuration, e.g., for engaging with a tissue surface.

FIGS. 8A-8B illustrate an embodiment of a cap (855) configured to fit over endoscope (850), wherein the cap (855) includes one or more protrusions (895) or keying features for aligning cap (855) with endoscope (850) and/or for forming a friction fit or seal against an outer surface of endoscope (850). The protrusions (895) may form an integral part of cap (855), or may comprise elements coupled to the cap (855), e.g., elastomeric rings for forming a seal with endoscope (850). As shown in FIG. 8B, cap (855) may include an elongated portion (890) such as a pull-on tab to facilitate placement of the cap (855) over the endoscope (855).

In another embodiment illustrated in FIG. 9B, cap (955) may have a tapered shape, wherein the outer diameter of the cap (955) narrows from a distal portion (965) to a proximal portion (960). For example, the outer diameter of the proximal portion (960) may be smaller than the outer diameter of the distal portion (965) such that the proximal portion (960) forms a friction fit against an endoscope. At least a portion of the proximal portion (960) may include a flexible material such that the proximal portion (960) may be pulled back or rolled up for placing the cap (955) around an endoscope, and then pulled down to form a seal around the endoscope.

Any of the features discussed herein in connection to an embodiment may be used in combination with one or more features of any other embodiment. Further, other embodiments of the present disclosure will be apparent to those skilled in the art from consideration of the specification and practice of the embodiments disclosed herein. It is intended that the specification and examples be considered as exemplary only, with a true scope of the present invention being indicated by the following claims.

## Claims

1. A medical device comprising:
a tube (350, 850) having a proximal end, a distal end, and at least one channel extending therebetween, the at least one channel in communication with a side aperture at the distal end of the tube (350, 850); and
a cap (355, 455, 555, 755, 855, 955) disposed on the distal end of the tube (350, 850), the cap (355, 455, 555, 755, 855, 955) including an opening in communication with the aperture and an expandable appendage (360, 460, 760) disposed around the opening, wherein the appendage (360, 460, 760) is configured to form a seal with a tissue surface, wherein a distal most surface of the appendage (360, 460, 760) includes a surface feature to grip the tissue surface.

2. The medical device of claim 1, wherein the appendage (360, 460, 760) includes an elastomeric material.

3. The medical device of claim 1, wherein the cap (355, 455, 555, 755, 855, 955) has a retracted configuration for moving the medical device along a body lumen and an expanded, conical configuration for engaging the appendage (360, 460, 760) with the tissue surface.

4. The medical device of claim 3, wherein the appendage includes doors (560) that pivot outward from the opening to engage the tissue surface.

5. The medical device of claim 1, wherein the cap (355, 455, 555, 755, 855, 955) is transparent.

6. The medical device of claim 1, wherein a distal most surface of the appendage (360, 460, 760) includes a deformable portion capable of forming a seal with the tissue surface.

7. The medical device of claim 1, wherein the cap (355, 455, 555, 755, 855, 955) is removable from the tube (350, 850).

8. The medical device of claim 1, further comprising at least one treatment instrument slidably disposed in the channel.

9. The medical device of claim 1, wherein the appendage (360, 460, 760) includes at least one inflatable member.

10. The medical device of claim 9, wherein the appendage includes a flexible membrane (660) and a plurality of inflatable members (665) attached to the membrane (660), wherein the inflatable members (665) extend radially outward from the opening to expand the membrane (660) into the conical shape.

11. The medical device of claim 1, wherein the medical device is configured for accessing the pancreaticobiliary system,
wherein the tube (350, 850) and the cap (355, 455, 555, 755, 855, 955) together form a suction device which has a retracted configuration for moving along a body lumen and an expanded configuration to form a seal with the tissue surface, wherein the tissue surface includes a papilla (106).

12. The medical device of claim 11, wherein the medical device further comprises a guidewire (310) to assist in introducing an instrument into at least a portion of the papilla (106).

13. The medical device of claim 12, wherein the instrument is a sphincterotome, the sphincterotome comprises a cutting wire for cutting at least a portion of the tissue surface.

14. The medical device of claim 1, wherein the cap (355, 455, 555, 755, 855, 955) includes an inflatable member on the back side opposite the expandable appendage (360, 460, 760).

## Patentansprüche

1. Medizinprodukt, das aufweist:
einen Schlauch (350, 850) mit einem proximalen Ende, einem distalen Ende und mindestens einem sich dazwischen erstreckenden Kanal, wobei der mindestens eine Kanal in Kommunikation mit einem Seitendurchlass am distalen Ende des Schlauchs (350, 850) steht; und
eine Kappe (355, 455, 555, 755, 855, 955), die auf dem distalen Ende des Schlauchs (350, 850) angeordnet ist, wobei die Kappe (355, 455, 555, 755, 855, 955) eine Öffnung in Kommunikation mit dem Durchlass und einen expandierbaren Anhang (360, 460, 760) aufweist, der um die Öffnung angeordnet ist, wobei der Anhang (360, 460, 760) so konfiguriert ist, dass er eine Dichtung mit einer Gewebeoberfläche bildet, wobei eine distalste Oberfläche des Anhangs (360, 460, 760) ein Oberflächenmerkmal aufweist, um die Gewebeoberfläche zu ergreifen.

2. Medizinprodukt nach Anspruch 1, wobei der Anhang (360, 460, 760) ein elastomeres Material aufweist.

3. Medizinprodukt nach Anspruch 1, wobei die Kappe (355, 455, 555, 755, 855, 955) eine eingezogene Konfiguration zum Bewegen des Medizinprodukts entlang eines Körperlumens und eine expandierte, konische Konfiguration zum Herstellen eines Eingriffs des Anhangs (360, 460, 760) mit der Gewebeoberfläche hat.

4. Medizinprodukt nach Anspruch 3, wobei der Anhang Klappen (560) aufweist, die von der Öffnung nach außen schwenken, um einen Eingriff mit der Gewebeoberfläche herzustellen.

5. Medizinprodukt nach Anspruch 1, wobei die Kappe (355, 455, 555, 755, 855, 955) transparent ist.

6. Medizinprodukt nach Anspruch 1, wobei eine distalste Oberfläche des Anhangs (360, 460, 760) einen verformbaren Abschnitt aufweist, der eine Dichtung mit der Gewebeoberfläche bilden kann.

7. Medizinprodukt nach Anspruch 1, wobei die Kappe (355, 455, 555, 755, 855, 955) vom Schlauch (350, 850) entfernbar ist.

8. Medizinprodukt nach Anspruch 1, das ferner mindestens ein Behandlungsinstrument aufweist, das im Kanal verschiebbar angeordnet ist.

9. Medizinprodukt nach Anspruch 1, wobei der Anhang (360, 460, 760) mindestens ein aufblasbares Teil aufweist.

10. Medizinprodukt nach Anspruch 9, wobei der Anhang eine flexible Membran (660) und mehrere aufblasbare Teile (665) aufweist, die an der Membran (660) angebracht sind, wobei sich die aufblasbaren Teile (665) von der Öffnung radial nach außen erstrecken, um die Membran (660) in die konische Form zu expandieren.

11. Medizinprodukt nach Anspruch 1, wobei das Medizinprodukt zum Zugang zum pankreatikobiliären System konfiguriert ist,
wobei der Schlauch (350, 850) und die Kappe (355, 455, 555, 755, 855, 955) gemeinsam eine Saugvorrichtung bilden, die eine eingezogene Konfiguration zum Bewegen entlang eines Körperlumens und eine expandierte Konfiguration hat, um eine Dichtung mit der Gewebeoberfläche zu bilden, wobei die Gewebeoberfläche eine Papille (106) aufweist.

12. Medizinprodukt nach Anspruch 11, wobei das Medizinprodukt ferner einen Führungsdraht (310) aufweist, um beim Einführen eines Instruments in mindestens einen Abschnitt der Papille (106) zu unterstützen.

13. Medizinprodukt nach Anspruch 12, wobei das Instrument ein Sphinkterotom ist, wobei das Sphinkterotom einen Schneiddraht zum Schneiden mindestens eines Abschnitts der Gewebeoberfläche aufweist.

14. Medizinprodukt nach Anspruch 1, wobei die Kappe (355, 455, 555, 755, 855, 955) ein aufblasbares Teil auf der Rückseite entgegengesetzt zum expandierbaren Anhang (360, 460, 760) aufweist.

## Revendications

1. Dispositif médical comprenant:
un tube (350, 850) ayant une extrémité proximale, une extrémité distale et au moins un canal s'étendant entre elles, le au moins un canal en communication avec une ouverture latérale à l'extrémité distale du tube (350, 850); et
un capuchon (355, 455, 555, 755, 855, 955) disposé sur l'extrémité distale du tube (350, 850), le capuchon (355, 455, 555, 755, 855, 955) incluant une ouverture en communication avec l'ouverture et un accessoire expansible (360, 460, 760) disposé autour de l'ouverture, où l'accessoire (360, 460, 760) est configuré pour former un contact étanche avec une surface tissulaire, où une surface la plus distale de l'accessoire (360, 460, 760) inclut une caractéristique de surface pour saisir la surface tissulaire.

2. Dispositif médical selon la revendication 1, où l'accessoire (360, 460, 760) inclut un matériau élastomère.

3. Dispositif médical selon la revendication 1, où le capuchon (355, 455, 555, 755, 855, 955) a une configuration rétractée pour déplacer le dispositif médical le long d'une lumière corporelle et une configuration conique expansée pour faire entrer en contact l'accessoire (360, 460, 760) avec la surface tissulaire.

4. Dispositif médical selon la revendication 3, où l'accessoire inclut des portes (560) qui pivotent vers l'extérieur depuis l'ouverture pour entrer en contact avec la surface tissulaire.

5. Dispositif médical selon la revendication 1, où le capuchon (355, 455, 555, 755, 855, 955) est transparent.

6. Dispositif médical selon la revendication 1, où une surface la plus distale de l'accessoire (360, 460, 760) inclut une partie déformable capable de former un contact étanche avec la surface tissulaire.

7. Dispositif médical selon la revendication 1, où le capuchon (355, 455, 555, 755, 855, 955) peut être retiré du tube (350, 850).

8. Dispositif médical selon la revendication 1, comprenant en outre au moins un instrument de traitement disposé de manière coulissante dans le canal.

9. Dispositif médical selon la revendication 1, où l'accessoire (360, 460, 760) inclut au moins un élément gonflable.

10. Dispositif médical selon la revendication 9, où l'accessoire inclut une membrane flexible (660) et une pluralité d'éléments gonflables (665) fixés à la membrane (660), où les éléments gonflables (665) s'étendent radialement vers l'extérieur depuis l'ouverture pour expanser la membrane (660) dans la forme conique.

11. Dispositif médical selon la revendication 1, où le dispositif médical est configuré pour accéder au système pancréaticobiliaire,
où le tube (350, 850) et le capuchon (355, 455, 555, 755, 855, 955) forment ensemble un dispositif d'aspiration qui a une configuration rétractée pour se déplacer le long d'une lumière corporelle et une configuration expansée pour former un contact étanche avec la surface tissulaire, où la surface tissulaire inclut une papille (106).

12. Dispositif médical selon la revendication 11, où le dispositif médical comprend en outre un fil de guidage (310) pour aider à introduire un instrument dans au moins une partie de la papille (106).

13. Dispositif médical selon la revendication 12, où l'instrument est un sphinctérotome, le sphinctérotome comprend un fil de coupe pour couper au moins une partie de la surface tissulaire.

14. Dispositif médical selon la revendication 1, où le capuchon (355, 455, 555, 755, 855, 955) inclut un élément gonflable sur le côté dorsal opposé à l'accessoire expansible (360, 460, 760).
